# EUROPEAN PATENT APPLICATION

(11) **EP 4 382 140 A1**
(43) Date of publication of application: **12.06.2024**
(21) Application number: 23214867.6
(22) Date of filing: 07.12.2023
(51) Int. Cl.: A61L 9/04, A61K 8/81, A61Q 13/00, C11D 3/50, A61L 9/01

(54) **FRAGRANCE COMPOSITION AND PREPARING METHOD THEREOF**

(30) Priority: 07.12.2022 US 202263386416 P
(71) Applicant: The Yankee Candle Company, Inc., South Deerfield, MA 01373 (US)
(72) Inventor: PALAFOX, Patrick, Longmeadow, MA 01106 (US); CAGLE, John, Southampton, MA 01073 (US); LIVELY, Christina, Rowe, MA 01367 (US)
(74) Representative: Cabinet Beau de Loménie

(57) **Abstract**

Provided herein are compositions containing a polymer substrate and a fragrance, methods for preparing such compositions, and methods for using such compositions for providing scents to an environment, such as a room or a vehicle. For example, the polymer substrate may comprise an alkyl acrylate, including but not limited to methyl acrylate. For example, the fragrance may comprise a polar or nonpolar molecule, or a collection of polar and/or nonpolar molecules. The resulting composition provides a temperature-stable fragrance composition that can be used within vehicles, has low to no residual odor, and can be loaded with at least 90% by weight of fragrance (relative to the weight of the polymer substrate).

## Description

### CROSS-REFERENCE TO RELATED APPLICATIONS

This application claims the benefit of priority from U.S. Provisional Patent Application 63/386,416 filed on December 7, 2022, the disclosure of which is incorporated herein by reference in its entirety.

### BACKGROUND OF INVENTION

Fragrance dispenser systems are generally provided as either active or passive systems. Active systems rely upon an external source of energy (e.g., heat) to activate fragrance for diffusion and/or a mechanism to dispense the fragrance into the surrounding environment (e.g., a fan or a spray mechanism). In comparison, passive systems typically do not rely on an external energy source and can diffuse the fragrance into air upon being exposed to the atmosphere. Regardless of the type of system used, the fragrances used with the fragrance dispenser system may be "loaded" into, stored within, or encapsulated within a solid, liquid, or gas fragrance carrier within the system.

Consumers' sense of smell presents a huge challenge for manufacturers of fragrance dispenser systems. What consumers perceive as a "scent" of a substance arises from a specialized set of sensory cells within the consumers' nasal cavities. When the substance is vaporized or aerosolized, some of the molecules comprising the substance can travel to these sensory cells. The sensory cells activated by the molecules (and thus the scents perceived by the consumers) depend upon the molecules' polarity, size, three-dimensional conformation, and other factors. Furthermore, what consumers perceive as a single smell may be a collection of hundreds or thousands of molecules detected by the sensory cells in tandem. Thus, while fragrances used with the fragrance dispenser systems may comprise a single molecule, in many cases a collection of molecules is used within the systems.

In light of the aforementioned challenges, to develop an effective fragrance dispenser system, the manufacturers seek a fragrance carrier capable of being loaded with hundreds or thousands of molecules-each molecule with its own unique chemical and physical properties. For solid-state fragrance carriers, manufacturers have turned to polymers, as polymers can, in certain circumstances, be loaded with fragrance molecules.

Ethylene vinyl acetate and its derivatives ("EVA") are polymers typically used in fragrance diffuser systems because EVA can hold onto many fragrance molecules with a high binding affinity. Unfortunately, EVA is not compatible with all types of fragrance molecules. Further, EVA binds to many fragrance molecules too tightly, which inhibits the molecules' release from the EVA. When the fragrance carrier binds to the fragrance molecules too tightly, the resulting fragrance composition will have a weak or non-existent smell.

Moreover, EVA is not an efficient carrier agent for fragrances. It can take a substantial amount of time to load EVA polymers with fragrance, representing a significant expense for fragrance manufacturers. Further, EVA will only release fragrance molecules from surfaces that are exposed to the outside environment and does not release the fragrance molecules retained within its body in appreciable amounts. Thus, much of the fragrance loaded into EVA is wasted, since many or most of the fragrance molecules remain trapped within the body of the polymer. This is particularly problematic for consumers and product manufacturers because fragrance molecules are typically the most expensive component of a fragrance dispenser system. Moreover, because the fragrance molecules are only released from EVA's surface, EVA-based compositions deliver a strong or "punchy" fragrance when the fragrance composition is first exposed to the atmosphere, but said compositions rapidly lose their perceived potency.

Given the problems presented by using EVA, manufacturers have attempted to develop a replacement carrier agent. Unfortunately, many potential solutions are not viable because the resulting polymers: have a high residual odor (which can overwhelm the scent produced by the fragrance molecules); are not compatible with the myriad of fragrance molecules desired by consumers; and/or are colored compounds which cannot be mixed with dyes to produce a product that is visually pleasing to consumers.

Thus, the market calls for a polymer carrier that is compatible with all fragrant molecules, can be loaded with a large proportion of fragrant molecules, is colorless, and allows for faster absorption, migration, and loading of fragrances than current polymers.

### SUMMARY OF INVENTION

The present invention overcomes many of the shortcomings and limitations of the prior art devices discussed above.

In one aspect, a fragrance composition is provided, the fragrance composition comprising a polymer substrate and a fragrance. The polymer substrate may comprise a reaction product of an acrylate monomer and an oligomer crosslinker, wherein the oligomer crosslinker has at least two arms. The fragrance may be encapsulated within the polymer substrate. In some embodiments, the acrylate monomer may include an alkyl functional group having 1 to about 10 carbon atoms. In other embodiments, the oligomer crosslinker may be provided as an acrylate oligomer including at least two acrylate functional groups.

In another aspect, a method of preparing a fragrance composition is provided. The method may comprise: (1) a dissolution step, wherein the monomer and the oligomer crosslinker are dissolved in an organic solvent, thereby forming a monomer-crosslinker solution; (2) a polymerization step, wherein the monomer is contacted with the oligomer crosslinker in the presence of an initiator to form a polymer substrate; and (3) a fragrance encapsulation step, wherein a fragrance is introduced to and encapsulated in the polymer substrate. Optionally, the method may further comprise (4) an extraction step, wherein the polymer substrate is extracted from the reaction mixture via an organic solvent bath, and/or (5) a washing step wherein the polymer substrate is purified.

In yet another aspect, a method of using the fragrance composition is provided. The method may comprise: (1) obtaining a fragrance composition comprising a polymer substrate and a fragrance; (2) positioning the fragrance composition in an environment; and (3) exposing the fragrance composition to the atmosphere in the environment.

These and other aspects and advantages of the present invention will become apparent to those skilled in the art after considering the following detailed description in connection with the accompanying drawings.

### BRIEF DESCRIPTION OF THE DRAWINGS

Fig. 1A illustrates a simplified chemical structure of an example polymer substrate produced via the methods disclosed herein, the polymer substrate imparted with a high molecular weight backbone and a low proportion of crosslinking;
Fig. 1B illustrates a simplified chemical structure of an example polymer substrate produced via the methods disclosed herein, the polymer substrate imparted with a high molecular weight backbone and a medium proportion of crosslinking;
Fig. 1C illustrates a simplified chemical structure of an example polymer substrate produced via the methods disclosed herein, the polymer substrate imparted with a high molecular weight backbone and a high proportion of crosslinking;
Fig. 2A illustrates a simplified chemical structure of an example polymer substrate produced via the methods disclosed herein, the polymer substrate imparted with a low molecular weight backbone and a low proportion of crosslinking;
Fig. 2B illustrates a simplified chemical structure of an example polymer substrate produced via the methods disclosed herein, the polymer substrate imparted with a low molecular weight backbone and a medium proportion of crosslinking; and
Fig. 2C illustrates a simplified chemical structure of two example polymer substrates produced via the methods disclosed herein, each polymer substrate imparted with a low molecular weight backbone and a high proportion of crosslinking.

### DETAILED DESCRIPTION

Provided herein are fragrance compositions containing a polymer substrate and a fragrance, methods for preparing such fragrance compositions, and methods for using such fragrance compositions. For example, provided herein are compositions including a polymer substrate comprising (a) a reaction product of an alkyl acrylate and a crosslinker comprising an acrylate oligomer, and (b) at least one fragrance molecule. Also provided herein are methods for preparing such compositions, and methods for using such compositions for delivering fragrances.

Advantageously, the compositions and methods provided herein may yield a fragrance composition having an improved product lifetime and a steady, increased intensity of fragrance release over the fragrance composition's lifetime. Further, the compositions and methods herein may provide a colorless polymer substrate with a low or nonexistent residual odor, and the polymer substrate may absorb a mass of fragrance molecules up to about 90% of the polymer substrate's weight. In addition, the compositions may provide a polymer substrate that can release fragrance molecules not only from surfaces exposed to the surrounding environment, but also from its body.

Further, in use cases beyond fragrance compositions, the methods provided herein may provide a polymer substrate that can absorb lubricants, plant-based oils, and/or synthetic oils. Moreover, the polymer substrate can act as a thickener, emulsifier, and/or a low-energy moldable replacement for thermoformed plastics. Finally, the polymer substrate may also modify the viscosity of aqueous-based systems.

### Definitions

As used herein, "AIBN" refers to the organic compound azobisisobutyronitrile, a compound with the chemical formula of ((CH₃)₂C(CN))₂N₂.

As used herein, the terms "alkyl" and "alkyl group" refer to a functional group having the chemical formula of CₙH₂ₙ₊₁, with n representing the number of carbon atoms in the functional group.

As used herein, the term "acrylate functional group" refers to a functional group with the chemical formula of CH₂=CHCOO⁻.

As used herein, the term "alkyl acrylate" refers to an organic compound comprising hydrogen or an alkyl group (R¹) bonded to an acrylate functional group. Generally, the alkyl acrylate compound may have the chemical formula of CH₂=CR²COO-R¹. R¹ may represent hydrogen or an alkyl group having between 1 carbon and about 10 carbon atoms. Preferably, R¹ represents an alkyl group having between 1 carbon and about 6 carbon atoms. In addition, R² may represent hydrogen or a methyl group.

As used herein, the term "arm" refers to a reaction site within a crosslinker wherein a monomer can chemically bond to the crosslinker.

As used herein, the term "colorless" refers to a substance without color. In some instances, the term colorless may be used to describe white substances.

As used herein, the term "encapsulate" refers to a polymer substrate binding a fragrance molecule, or a collection of fragrance molecules, within a body of the polymer substrate and on surfaces of the body.

As used herein, "EtOAc" refers to the organic compound ethyl acetate, a compound with the chemical formula of CH₃CO₂CH₂CH₃.

As used herein, the term "fragrance" refers to a single fragrance molecule, or a collection of fragrance molecules, that can produce a scent detectable by a living organism.

As used herein, "MA-TMPTMA" refers to a polymer produced by the polymerization reaction comprising, consisting essentially of, or consisting of methyl acrylate bonded to TMPTMA. The MA-TMPTMA polymer may have the general chemical structure of 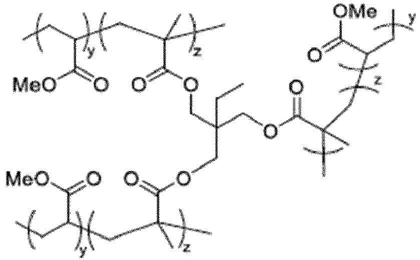 wherein "y" and "z" within the structural formula are average numbers greater than 1.

As used herein, "MeOH" refers to the organic compound methanol, a compound with the formula CH₃OH.

As used herein, "TMPTMA" refers to an oligomer, namely trimethylolpropane trimethacrylate, that may be utilized as a crosslinker in a polymerization reaction. TMPTMA has the linear chemical formula of [H₂C=C(CH₃)CO₂CH₂]CC₂H₅.

### Compositions

Provided herein are fragrance compositions comprising (1) a polymer substrate and (2) a fragrance.

The polymer substrate may comprise, consist essentially of, or consist of a monomer bound to an oligomer crosslinker. Alternatively, the polymer substrate may comprise a reaction product of a monomer and a crosslinker comprising an acrylate oligomer. Generally, the monomer may have the following chemical structure: 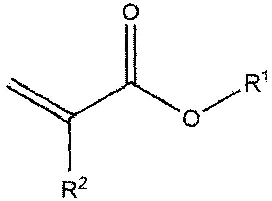 R¹ is selected from the group consisting of hydrogen and C₁-C₁₀ alkyl groups, and R² is selected from the group consisting of H and CH₃. For example, R¹ may comprise a methyl group and R² may comprise hydrogen. As an additional example, R¹ may comprise an ethyl group and R² may comprise a methyl group.

Non-limiting examples of the monomers include acrylate-containing compounds, for example, alkyl acrylates, methyl acrylate, ethyl acrylate, propyl acrylates, butyl acrylates, pentyl acrylates, hexyl acrylates, and mixtures thereof.

The oligomer crosslinker preferably comprises TMPTMA. Generally, the oligomer crosslinker may comprise, consist essentially of, or consist of an acrylate-containing oligomer. For example, the oligomer crosslinker may comprise, consist essentially of, or consist of an acrylate oligomer derived from trimethylolpropane. For example, the oligomer crosslinker may comprise an at least two-armed acrylate oligomer (i.e., a crosslinker with at least two bonding sites for a monomer). For example, the oligomer crosslinker may comprise a threearmed acrylate crosslinker (i.e., a crosslinker with three bonding sites for a monomer).

The fragrance that can be used in the fragrance composition is not particularly limited and may include polar and/or nonpolar compounds. By way of non-limiting example, the fragrance may comprise, consist essentially of, or consist of an ester-containing compound, a terpene-containing compound, an aromatic-containing compound, an amine-containing compound, an alcohol-containing compound, an aldehyde-containing compound, a ketonecontaining compound, a lactone-containing compound, a thiol-containing compound, a phosphine-containing compound, a diacetyl-containing compound, a hydrothiophene-containing compound, a pyrazine-containing compound, and/or combinations thereof. By way of another non-limiting example, the fragrance may comprise a molecule or a collection of molecules that are detectable as a scent by a living organism (e.g., a human).

The fragrance composition may comprise the polymer substrate and the fragrance. The methods herein may produce a composition with a fragrance to polymer substrate mass ratio of at least about 5:1, at least about 4:1, or at least about 3:1, or at least about 2:1, or at least about 1:1, or at least about 2:3, or at least about 1:2, or at least about 1:3. Alternatively, the methods herein may produce a composition with a fragrance to polymer substrate mass ratio of at most about 1:3, or at most about 1:2, or at most about 1:1, or at most about 2:1.

For example, the fragrance composition may comprise the fragrance in an amount of at least about 20% (w/w), or at least about 25% (w/w), or at least about 30 % (w/w), or at least about 35% (w/w), or at least about 40% (w/w), or at least about 45% (w/w), or at least about 50% (w/w), or at least about 55% (w/w), or at least about 60% (w/w), or at least about 65% (w/w), or at least about 70% (w/w), or at least about 75% (w/w), or at least about 80% (w/w). Alternatively, the fragrance composition may comprise the fragrance in an amount of at most about 70% (w/w), or at most about 65% (w/w), or at most about 60% (w/w), or at most about 50% (w/w), or at most about 40% (w/w), or at most about 30% (w/w).

For example, the fragrance composition may comprise the polymer substrate in an amount of at least about 20% (w/w), or at least about 30% (w/w), at least about 35% (w/w), or at least about 40% (w/w), or at least about 45% (w/w), or at least about 50% (w/w), or at least about 55% (w/w), or at least about 60% (w/w), or at least about 65% (w/w), or at least about 70% (w/w), or at least about 75% (w/w). Alternatively, the fragrance composition may comprise the polymer substrate in an amount of at most about 80% (w/w), or at most about 75% (w/w), or at most about 70% (w/w), or at most about 65% (w/w), or at most about 60% (w/w), or at most about 55% (w/w), or at most about 50% (w/w), or at most about 45% (w/w), or at most about 40% (w/w).

Additionally, the concentration of fragrance in the fragrance composition may fall within a range defined by any of the values listed above. For example, the fragrance composition may comprise the fragrance in an amount of about 20% to about 80% (w/w), or about 30% to about 70% (w/w), or about 40% to about 60% (w/w). As an additional example, the fragrance composition may comprise the fragrance to polymer substrate in a mass ratio of about 3:1 to about 1:3, or about 2:1 to about 1:2, or about 1.5:1 to about 1:1.5.

Additionally, the concentration of the polymer substrate in the fragrance composition may fall within a range defined by any of the values listed above. For example, the fragrance composition may comprise the polymer substrate in an amount of about 25% to about 85% (w/w), or about 35% to about 65% (w/w), or about 45% to about 60% (w/w). As an additional example, the fragrance composition may comprise the polymer substrate to fragrance in a mass ratio of about 3:1 to about 1:3, or about 2:1 to about 1:2, or about 1.5:1 to about 1:1.5.

The polymer substrate may be produced via a chemical reaction, e.g., a polymerization reaction. Generally, as explained in greater detail below, reactants and/or reagents of the chemical reaction may comprise, consist essentially of, or consist of the monomer, the oligomer crosslinker, an initiator, and/or a solvent. For example, the reactants of the chemical reaction may comprise the monomer and the oligomer crosslinker.

The polymer substrate may comprise a reaction product of the oligomer crosslinker and the monomer, wherein the oligomer crosslinker and the monomer are combined in a mass ratio of at least about 1:1, at least about 1:2, or at least about 1:3, or at least about 1:5, or at least about 1:10, or at least about 1:15, or at least about 1:20, or at least about 1:25, or at least about 1:30, or at least about 1:35, or at least about 1:40 to produce the polymer substrate. Alternatively, the polymer substrate may comprise a reaction product of the oligomer crosslinker and the monomer, wherein the oligomer crosslinker and the monomer are combined in a mass ratio of at most about 1:50, or at most about 1:40, or at most about 1:30, or at most about 1:25, or at most about 1:20, or at most about 1:15, or at most about 1:10, or at most about 1:5 to produce the polymer substrate.

For example, the polymer substrate may comprise a reaction product of the oligomer crosslinker and the monomer, wherein the oligomer crosslinker and the monomer are combined in a mole ratio of at least about 1:5, or at least about 1:7, or at least about 1:10, or at least about 1:20, or at least about 1:30, or at least about 1:50, or at least about 1:100, or at least about 1:150 to produce the polymer substrate. Alternatively, the oligomer crosslinker and the monomer are combined in a mole ratio of at most about 1:200, or at most about 1:150, or at most about 1:100, or at most about 1:75, or at most about 1:50, or at most about 1:30, or at most about 1:20, or at most about 1:10 to produce the polymer substrate.

Additionally, the concentration of the oligomer crosslinker and the monomer may be combined within a range defined by any of the values listed above. For example, the oligomer crosslinker and the monomer may be combined in a mass ratio from about 1:2 to about 1:50, or from about 1:5 to about 1:40, or from about 1:10 to about 1:30, or from about 1:15 to about 1:25 to produce the polymer substrate. As an additional example, the oligomer crosslinker and the monomer may be combined in a mole ratio from about 1:5 to about 1:200, or from about 1:10 to about 1:150, or from about 1:30 to about 1:100, or from about 1:50 to about 1:75 to produce the polymer substrate.

### Preparation Methods

Provided herein are methods for preparing a fragrance composition comprising a polymer substrate and a fragrance (e.g., the composition described above). The methods may comprise, consist essentially of, or consist of any one or more of the following: (1) a dissolution step, wherein a monomer and an oligomer crosslinker are dissolved in an organic solvent, thereby forming a monomer-crosslinker solution; (2) a polymerization step, wherein the monomer is contacted with the oligomer crosslinker in the presence of an initiator to form a polymer substrate; and (3) a fragrance encapsulation step, wherein a fragrance is introduced to and encapsulated in the polymer substrate.

Optionally, the initiator may be introduced to the reaction mixture during the dissolution step. Optionally, or alternatively, the initiator may be introduced to the reaction mixture during the polymerization step.

Optionally, the initiator may be consumed as a reactant in the polymerization reaction forming the polymer substrate, thereby forming a portion of the polymer substrate. Optionally, or alternatively, the initiator may be provided as a reagent such that the initiator does not form a portion of the polymer substrate.

Optionally, the methods may also comprise: (4) an extraction step, wherein the polymer substrate is extracted from the reaction mixture via an organic solvent bath; (5) a washing step wherein the polymer substrate is purified; and (6) a drying step wherein moisture is removed from the polymer substrate.

Optionally, the purity of the polymer substrate may be determined after the washing step is complete. In some instances, the purity of the polymer substrate may be determined after the washing step and the drying step are complete.

### Dissolution Step

The methods provided herein may comprise a dissolution step wherein a monomer and an oligomer crosslinker are dissolved in an organic solvent, thereby forming a monomer-crosslinker solution.

Preferably, the organic solvent is a polar organic solvent. The organic solvent may comprise, consist essentially of, or consist of a polar organic solvent. Non-limiting examples include acetate-containing solvents, alcohol-containing solvents, sulfoxide-containing solvents, formamide-containing solvents, carbonate-containing solvents, and the like. Preferred solvents include alkyl acetates and mixtures thereof. A particularly preferred solvent is ethyl acetate (EtOAc).

Optionally, the monomer, the oligomer crosslinker, and the organic solvent are mixed during the dissolution step. The mixing may be carried out by an apparatus that can physically combine the monomer, the oligomer crosslinker, and the organic solvent. For example, the apparatus may comprise a magnetic stirrer, a static mixer (e.g., a ribbon mixer), an in-line mixer, a drum mixer, a planetary mixer, an agitator, a continuous mixer, and the like.

### Polymerization Step

The methods provided herein may comprise a polymerization step wherein the monomer is reacted with a crosslinker in the presence of an initiator, thereby forming a polymer substrate.

The initiator may comprise, consist essentially of, or consist of a radical-type initiator. Non-limiting examples of radical initiators include nitrogen-halogen initiators, azo compounds, organic peroxides, and inorganic peroxides. For example, the initiator may comprise, consist essentially of, or consist of an azo-type initiator. Preferably, the initiator may be provided as AIBN.

Optionally, the initiator may comprise a portion of the reagents and/or the reactants used to form the polymer substrate. The reagents and/or reactants provided to form the polymer substrate may comprise the initiator, as compared to the oligomer crosslinker, in a mass ratio of at least about 1:1, or at least about 1:2, or at least about 1:3, or at least about 1:4, or at least about 1:6, or at least about 1:8. Alternatively, the reagents and/or reactants provided to form the polymer substrate may comprise the initiator, as compared to the oligomer crosslinker, in a mass ratio of at most about 1:10, or at most about 1:7, or at most about 1:5, or at most about 1:4, or at most about 1:3, or at most about 1:2.

Alternatively, the reagents and/or reactants provided to form the polymer substrate may comprise the initiator, as compared to the oligomer crosslinker, in a mole ratio of at least about 1:2, or at least about 1:3, or at least about 1:4, or at least about 1:5, or at least about 1:6. Alternatively, the reagents and/or reactants provided to form the polymer substrate may comprise the initiator, as compared to the oligomer crosslinker, in a mole ratio of at most about 1:8, or at most about 1:6, or at most about 1:4.

Additionally, the reagents and/or reactants provided to form the polymer substrate may comprise the initiator, as compared to the oligomer crosslinker, within a range defined by any of the values listed above. For example, the reagents and/or reactants provided to form the polymer substrate may comprise the initiator, as compared to the oligomer crosslinker, in a mass ratio from about 1:1 to about 1:10, or from about 1:2 to about 1:8, or from about 1:3 to about 1:7, or from about 1:4 to 1:6. As an additional example, the reagents and/or reactants provided to form the polymer substrate may comprise the initiator, as compared to the oligomer crosslinker, in a mole ratio from about 1:2 to about 1:6, or from about 1:3 to about 1:5.

Optionally, the polymerization reaction may be carried out after a mixture comprising, consisting essentially of, or consisting of the initiator, the monomer, and the oligomer crosslinker mixture is purged with an inert gas. Non-limiting examples of suitable inert gases include nitrogen gas (N_{2(g)}), carbon dioxide gas (CO_{2(g)}), argon gas (Ar_{(g)}), helium gas (He_{(g)}), and mixtures thereof. The purging may be carried out for a first predetermined time. For example, the first predetermined time may be at least about 10 minutes, or at least about 15 minutes, or at least about 30 minutes, or at least about 45 minutes, or at least about 1 hour. Alternatively, the first predetermined time may be from about 15 minutes to about 1 hour.

Optionally, the polymerization reaction may be carried out for a second predetermined time. For example, the second predetermined time may be at least about 1 hour, or at least about 1.25 hours, or at least about 1.5 hours, or at least about 1.75 hours, or at least about 2 hours, or at least about 2.25 hours, or at least about 2.5 hours, or at least about 3 hours. Alternatively, the second predetermined time may be from at least about 1 hour to no more than about 3 hours.

Optionally, the polymerization step may be performed at a first elevated temperature. The polymerization step may be carried out at a temperature of at least about 40°C, or at least about 50°C, or at least about 60°C, or at least about 65°C, or at least about 70°C, or at least about 75°C, or at least about 80°C, or at least about 90°C, or at least about 100°C. For example, the polymerization step may be carried out at a temperature from about 50°C to about 90°C, from about 60°C to about 80°C, or from about 70°C to about 75°C.

Optionally, heat may be applied during the polymerization step for a third predetermined time. For example, the third predetermined time may be at least about 1 hour, or at least about 1.25 hours, or at least about 1.5 hours, or at least about 1.75 hours, or at least about 2 hours, or at least about 2.25 hours, or at least about 2.5 hours, or at least about 3 hours. Alternatively, the third predetermined time may be from at least about 1 hour to no more than about 3 hours. In some instances, the third predetermined time may be concurrent with the second predetermined time.

Optionally, the polymerization reaction may be quenched after the second and/or the third predetermined time. To quench the polymerization reaction, an organic quenching agent may be introduced to the reaction mixture. Non-limiting examples of suitable organic quenching agents include acetate-containing solvents, alcohol-containing solvents, sulfoxide-containing solvents, formamide-containing solvents, and carbonate-containing solvents. Preferred organic quenching agents include alkyl acetates and mixtures thereof. A particularly preferred organic quenching agent is MeOH. In some instances, the reaction product solution may be cooled to room temperature after the organic solution is introduced to the reaction product solution.

Generally, the primary product of the polymerization step will be the polymer substrate. In a preferred embodiment, the acrylate monomer comprises methyl acrylate, and the oligomer crosslinker comprises TMPTMA. When the methyl acrylate and the TMPTMA are provided, the polymerization step forms an MA-TMPTMA polymer substrate as shown in the reaction scheme below.

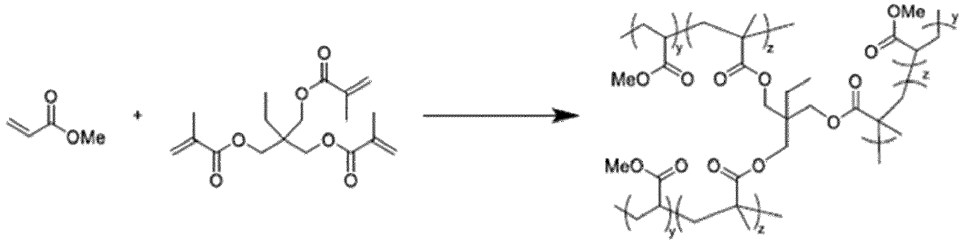

In alternative embodiments, a variety of alkyl substituents can be employed in the acrylate monomer to achieve the desired properties in the polymer substrate. It is generally preferred to utilize monomers having alkyl substituents containing at most about 10 carbon atoms. For example, the alkyl substituents may comprise an average of from 1 to about 6 carbon atoms.

It has been discovered that the polymer substrate produced in the polymerization step exhibits a number of surprising advantages. Importantly, the polymer substrate may be used to produce a fragrance composition that has a longer lifetime and increased scent intensity compared to prior art polymers, including EVA. Furthermore, the polymer substrate may be loaded with or encapsulate virtually any fragrance, including fragrances incompatible with EVA. In addition, the polymer substrate may be saturated with fragrance more quickly than EVA. Moreover, the polymer substrate may release fragrance not only from surfaces exposed to the outside environment but also from its body. Finally, the polymer substrate is colorless and has a low or non-existence residual odor.

### Fragrance Loading Step

The methods provided herein may comprise a fragrance encapsulation step wherein a fragrance is introduced to the bulk polymer substrate. The fragrance may be provided to the polymer substrate in the form of a solid, a liquid, or a gas.

Without being bound to a particular theory, it is believed that the fragrance molecules are physically absorbed within a body and/or on surfaces of the polymer substrate due to the porous nature of the polymer substrate. The porosity of the polymer substrate may be due to an open and/or branched chemical structure of the polymer substrate formed using the methods described herein. Once the fragrance is absorbed onto the surface and/or within the body of the polymer substrate, intermolecular forces may hold the fragrance within the polymer substrate.

### Extraction Step

The methods provided herein may comprise an extraction step, wherein the polymer substrate is extracted from the reaction mixture.

The extraction step may comprise distilling the reaction mixture. Preferably, the distillation occurs at a second elevated temperature and at a first reduced pressure. For example, the distillation may occur at a second elevated temperature of at least about 50°C, or at least about 60°C, or at least about 65°C, or at least about 70°C, or at least about 75°C, or at least about 80°C, or at least about 90°C, or at least about 100°C. For example, the polymerization step may be carried out at a temperature from about 50°C to about 90°C, from about 60°C to about 80°C, or from about 70°C to about 75°C. The first reduced pressure may be less than atmospheric pressure. For example, the first reduced pressure may be no more than about 1 atm, or no more than about 0.8 atm, or no more than about 0.6 atm, or no more than about 0.4 atm, or no more than about 0.2 atm, or no more than about 0.1 atm. Alternatively, the first reduced pressure may be from about 0.1 atm to about 1 atm, or from about 0.2 atm to about 0.8 atm, or from about 0.4 atm to about 0.6 atm.

The extraction step may comprise extracting the polymer substrate from the reaction mixture via an organic solvent bath. Preferably, an organic solvent used in the organic solvent bath comprises a polar organic solvent. Particularly, the polar organic solvent may comprise a mixture of methanol and acetone.

### Washing Step

The methods provided herein may comprise a washing step wherein the polymer substrate is purified. The washing step may include washing the polymer substrate with an organic solvent such that impurities (e.g., residuals) are removed from the polymer substrate. Preferably, the organic solvent comprises a mixture of acetone and MeOH.

Optionally, after the polymer substrate is washed with the organic solvent, the polymer substrate may be further purified via filtration and additional organic solvent washings. Preferably, the organic solvent used in the additional washings is MeOH.

### Drying Step

After washing of the polymer substrate is complete, the polymer substrate may be dried at a third elevated temperature and a second reduced pressure. For example, the third elevated temperature may be at least about 60°C, or at least about 65°C, or at least about 70°C, or at least about 75°C, or at least about 80°C, or at least about 90°C, or at least about 100°C. Alternatively, the third elevated temperature may be from about 50°C to about 90°C, from about 60°C to about 80°C, or from about 70°C to about 75°C. For example, the second reduced pressure may be no more than about 1 atm, or no more than about 0.8 atm, or no more than about 0.6 atm, or no more than about 0.4 atm, or no more than about 0.2 atm, or no more than about 0.1 atm. Alternatively, the second reduced pressure may be from about 0.1 atm to about 1 atm, or from about 0.2 atm to about 0.8 atm, or from about 0.4 atm to about 0.6 atm.

### Purity Analysis of Polymer Substrate

After washing is complete, the purity of the polymer substrate may be determined using a variety of analytical methods. For example, the purity of the polymer substrate may be determined via a thermogravimetric analysis of a sample of the polymer substrate.

Generally, the purity of the polymer substrate may be indicated by a residual percentage obtained from the thermogravimetric analysis. The residual percentage may indicate the weight percent of non-product residuals within the polymer substrate (e.g., moisture, solvent). Once the washing step is complete, the residual percentage (as determined by the thermogravimetric analysis) may be at most about 5% (w/w), or at most about 4% (w/w), or at most about 3% (w/w), or at most about 2.5% (w/w), or at most about 2% (w/w), or at most about 1.5% (w/w), or at most about 1% (w/w), or at most about 0.5% (w/w), or at most about 0% (w/w). For example, after the washing step, the residual percentage may be about 0% to about 5% (w/w), or about 0.5% to about 2.75% (w/w).

In some instances, the purity of the polymer substrate may be determined after a washing step that is followed by the drying step. Once the washing and drying steps are complete, the residual percentage (as determined by the thermogravimetric analysis) may be at most about 5% (w/w), or at most about 4% (w/w), or at most about 3% (w/w), or at most about 2.5% (w/w), or at most about 2% (w/w), or at most about 1.5% (w/w), or at most about 1% (w/w), or at most about 0.5% (w/w), or at most about 0% (w/w). For example, after the washing step including the optional drying is complete, the residual percentage may be about 0% to about 5% (w/w), or about 0.5% to about 2.75% (w/w).

Generally, the purity of the polymer substrate may be indicated by a percent purity value. After the washing step is complete (either with or without the optional drying step), the purity value of the polymer substrate may be at least about 90%, or at least about 92%, or at least about 94%, or at least about 95%, or at least about 96%, or at least about 97%, or at least about 98%, or at least about 99%, or at least about 100%.

### Polymer Substrate Forms

The inventors have surprisingly discovered that the polymer substrates described herein, and MA-TMPTMA polymer substrates in particular, may be used as a polymer substrate in the fragrance compositions described herein. The fragrance compositions may be incorporated into both active fragrance dispenser systems and passive fragrance dispenser systems. Non-limiting examples of fragrance products that may use the polymer substrates may include hanging dispensers, stick-on dispensers, reed dispensers, fragrance clips, fragrance screens, and the like.

The polymer substrate described herein can be produced with a low residual odor and is colorless. These characteristics allow for the production of a fragrance composition that is aesthetically and sensually pleasing to consumers. Because the polymer substrate has a low residual odor, consumers may not detect a scent associated with the polymer substrate, which allows the consumers to experience the scent of the fragrance in an unadulterated form. Further, because the polymer substrate is colorless, fragrance manufacturers may dye the fragrance compositions to produce a product that is pleasing to look at.

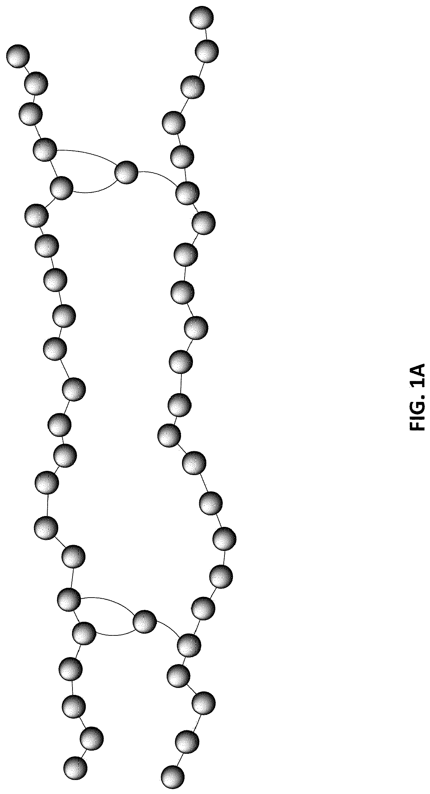

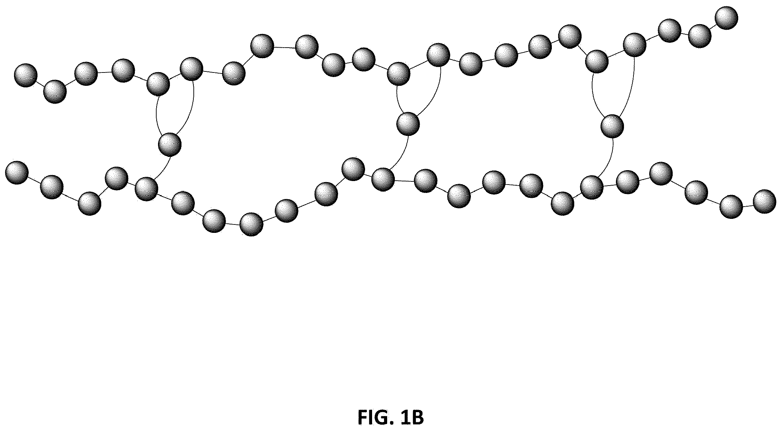

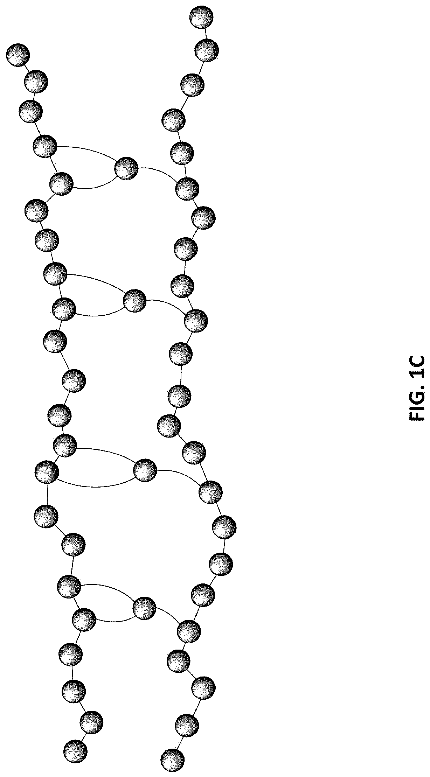

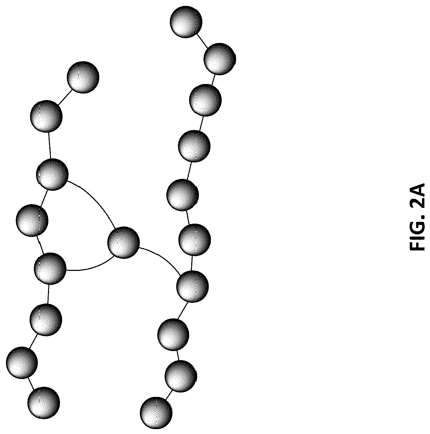

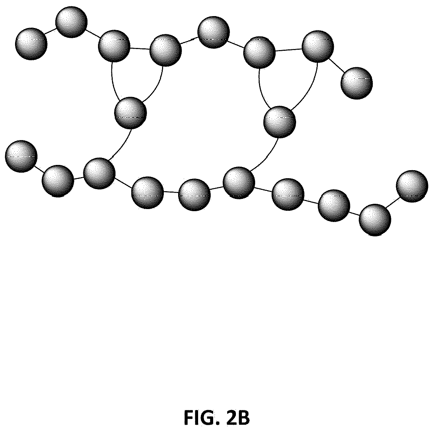

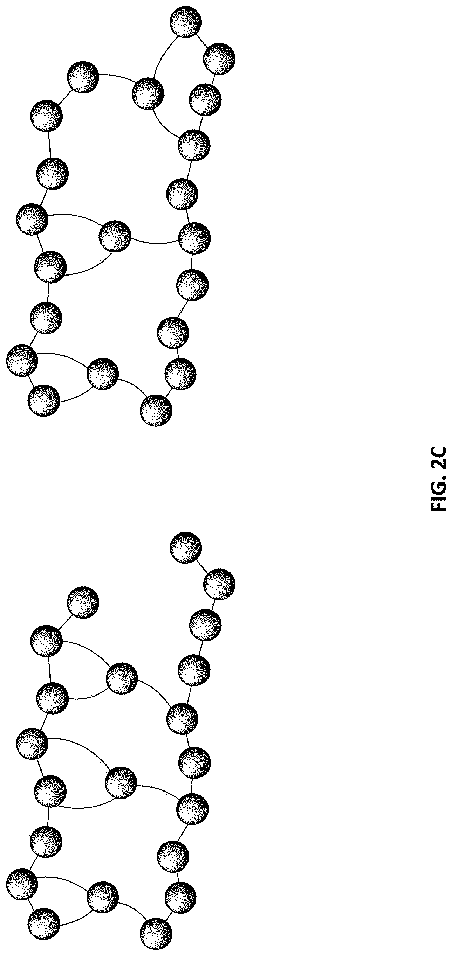

Without being bound to a particular theory, the functionality of the polymer can be related to the size of the polymer substrate's MA backbone (Fn-1) and the amount of crosslinker comprising the polymer substrate (Fn-2). The polymer substrate can be imparted with various Fn-1 and Fn-2 values using known methods. The polymer substrate can generally be incorporated into any solid or liquid state fragrance composition known in the art. As the Fn-1 and Fn-2 values of the polymer substrate are varied, the form of the polymer substrate (and thus the form of the fragrance composition created after the polymer substrate is loaded with fragrance) will also vary. For example, the polymer substrate can be in the form of a granulated powder, or gel. As further non-limiting examples, the composition can be in the form of a liquid suspension, emulsion, or aqueous solution. In addition, the polymer substrate may take the form of a tacky gel, a semi-solid, a thickening agent (i.e., viscosity modifier), an absorbent powder, or a super-absorbent powder. Example polymer substrates imparted with different Fn-1 and Fn-2 values are illustrated in FIGS. 1A-2C.

The fragrance compositions provided herein may be used to produce a fragrance product that is longer lasting and delivers a more consistent amount of fragrance over the compositions' lifetime. For example, the inventors have surprisingly discovered that the fragrance compositions provided herein may last two to four times longer than current products. Without being bound to a particular theory, it is believed that the open and/or branched structure of the polymer substrate allows for the migration of the fragrance from the body of the polymer substrate to the surfaces of the polymer substrate, which effectively increases the amount of fragrance that the fragrance composition can release over its lifetime compared to current products. This also may allow for the fragrance composition to provide a more consistent release of fragrance over its lifetime. Moreover, the fragrance composition herein provides for faster encapsulation of fragrances than current products.

The fragrance compositions provided herein may be used, for example, to deliver fragrance in environments including, but not limited to, rooms, buildings, vehicles, and the like. The inventors have surprisingly discovered that the compositions described herein are stable within high-temperature environments, such as those that may be found within vehicles. Particularly, the polymer substrate (and the associated fragrance compositions) are not prone to deformation at high temperatures. In addition, the fragrance compositions described herein may be provided in a variety of fragrance products, including, but not limited to, car jars, candles, electronic plugin devices, wall plugin devices, and the like.

### Examples

The following non-limiting examples are provided to further illustrate the present disclosure.

### Example Preparation Method

15 g (0.17 mol) of MA and 6.56 g (19.4 mmol) of TMPTMA were dissolved in 150 mL of EtOAc to create a pre-polymerized solution. 959 mg (5.80 mmol) of AIBN was dissolved in 50 mL of EtOAc and added to the pre-polymerized solution. The pre-polymerized solution was purged with gaseous nitrogen (N_{2(g)}) for 30 minutes, and then heated to 70°C and stirred for 2 hours under an inert atmosphere. After the 2 hours had elapsed, MeOH was used to quench the reaction and the resulting polymer substrate suspension was cooled to room temperature. After cooling, the polymer substrate suspension was distilled under reduced pressure at 70°C to separate the polymer substrate from the remaining components of the suspension. The recovered white solid (i.e., the polymer substrate) was cooled to room temperature and washed with acetone and MeOH. Then, the remaining solid was filtered and washed with additional MeOH. The recovered solid substrate was placed on reduced pressure at 80°C.

### Example uses of the polymer substrate based on appearance when subjected to various fragrance oils

The function of the polymer substrate can be related to the appearance or solubility of the polymer substrate when it is dosed to fragrances. Fragrance oils are designed specifically for the fragrance products' "end use"; thus, fragrance oils can be categorized by their designated end use. For example, EVA-type fragrance oils are designed to solubilize specific parts of an EVA polymer and then vaporize at ambient conditions. In comparison, fragrance oils with an end use in heated diffusers are designed to vaporize at elevated temperatures. The polymer substrates listed in Table 1 (i.e., the polymer substrates of Examples 1-17) were exposed to fragrance oils such that the fragrance oils comprised 50% (w/w) of the total mass of the fragrance composition. The appearance of the fragrance compositions varied per example and per fragrance oil type, as outlined in Table 1.

**Table 1**

| **Appearance at 50% (w/w) of Fragrance** | | | | | |
|---|---|---|---|---|---|
| **Example** | **Fragrance Type** | | | **TMPTMA (mol %)** | **AIBN (mol %)** |
| | **EVA** | **Reed Diffuser** | **Heated Diffuser** | | |
| 1 | A, D | A, D | | 3 | 1 |
| 2 | B, D | A, D | | 5 | 1 |
| 3 | B, D | | | 10 | 1 |
| 4 | A, D | B, D | | 3 | 2 |
| 5 | A, D | A, D | | 5 | 2 |
| 6 | B, D | B, D | B,C | 10 | 2 |
| 7 | A, C | | | 15 | 2 |
| 8 | A, D | | | 5 | 3 |
| 9 | B, D | | B, C | 10 | 3 |
| 10 | B, C | B, C | B, C | 15 | 3 |
| 11 | A, D | | | 5 | 5 |
| 12 | B, D | | | 10 | 5 |
| 13 | B, C | A, C | A, C | 15 | 5 |
| 14 | A, C | A, C | A, C | 20 | 5 |
| 15 | A, C | | | 30 | 5 |
| 16 | F | F | F | 5 | 7 |
| 17 | A, D | | 5 | | 10 |

| | | | | | |
|---|---|---|---|---|---|
| A = Swell: volume increase, B = Minimal Swelling: No visible increase in volume, C = Absorbed: No visible liquid is seen, polymer appears dry, D =Mostly Absorbed: Small amount of liquid is seen, possibly past the saturation point of the polymer substrate, E = Minimal Absorption: Majority of liquid is seen, F = Dissolved: Homogeneous solution, AIBN = 2,2'-Azobis(2-methylpropionitrile), TMPTMA = Trimethylolpropane trimethacrylate | | | | | |

The variation in appearance for the polymer substrates of Examples 1-17 was related to the composition of the fragrance oil and the composition of the polymer substrate (e.g., see FIGS. 1A-2C). To expand on the functionality of the polymer substrate, fragranced polymer examples that had a swollen appearance (see Table 1) were dosed with an increased quantity of fragrance oil, such that the fragrance comprised 70% (w/w) of the total mass of the sample. Table 2 demonstrates how by varying Fn-1 and Fn-2, the polymer substrate can absorb a relatively high amount of fragrance oil with noticeable swelling. To further show the polymer's functionality as a fragrance release substrate, Example 14, which showed a relatively high amount of absorbance with the EVA fragrance oil, was compared to other fragrance products designed to release fragrance under ambient conditions.

**Table 2**

| **Absorption at 70% (w/w) of Fragrance** | | | | | |
|---|---|---|---|---|---|
| **Example** | **Fragrance Type** | | | **TMPTMA (mol %)** | **AIBN (mol %)** |
| | **EVA** | **Reed Diffuser** | **Heated Diffuser** | | |
| 1 | D | D | | 3 | 1 |
| 2 | | D | | 5 | 1 |
| 5 | D | E | | 5 | 2 |
| 7 | C | | | 15 | 2 |
| 8 | D | | | 5 | 3 |
| 10 | C | D | D | 15 | 3 |
| 13 | C | D | D | 15 | 5 |
| 14 | C | C | C | 20 | 5 |
| 15 | C | | | 30 | 5 |
| 16 | E | | | 5 | 7 |
| 17 | E | | | 5 | 10 |

| | | | | | |
|---|---|---|---|---|---|
| C = Absorbed: No visible liquid is seen and polymer appears dry, D = Mostly Absorbed: Small amount of liquid is seen, possibly past the saturation point of the polymer substrate, E = Minimal Absorption: Majority of liquid is seen, F = Dissolved: Homogeneous solution, AIBN = 2,2'-Azobis(2-methylpropionitrile), TMPTMA = Trimethylolpropane trimethacrylate | | | | | |

The olfactory intensity comparison of samples consisting of fragranced Example 14 and presently available products were examined using a neutral room on an olfactory intensity scale of 0-7, with 7 representing the highest relative scent intensity. The examination and assignment of scent intensities were carried out via a trained panel of human testers. All of the samples used in the comparisons were monitored over 4 weeks, with the olfactory intensity recorded at various weeks. Table 3 outlines the fragrances used during the comparison, where the Auto Vent product fragrances were matched based on similar olfactory notes.

**Table 3**

| **Scented EVA** | | **Scented Polymer** | | **Scented Paper** | | **Auto Vent Product** | |
|---|---|---|---|---|---|---|---|
| **Ex. 14** | **Product** | **Ex. 14** | **Product** | **Ex. 14** | **Product** | **Ex. 14** | **Product** |
| Floral-1 | Floral-1 | Citrus-1 | Citrus-1 | Floral-1 | Floral-1 | Floral-1 | Floral-2 |
| Fresh-1 | Fresh-1 | Fresh-1 | Fresh-1 | Fresh-1 | Fresh-1 | Woody-1 | Woody-2 |
| | | | | | | Fresh-2 | Fresh-3 |

The olfactory intensity profiles for each of the samples versus a comparator EVA product are provided in Tables 4-7 below:

**Table 4**

| **Intensity Profile of Example 14 vs. EVA Product** | | | | | | | | | | |
|---|---|---|---|---|---|---|---|---|---|---|
| **Week** | **Trial 1** | | | | | **Trial 2** | | | | |
| | **Fragranced Sample¹** | | | **EVA Product²** | | **Fragranced Samples¹** | | | **EVA Product²** | |
| | **1** | **2** | **3** | **A** | **B** | **4** | **5** | **6** | **C** | **D** |
| **0** | - | - | - | - | - | 4.89 | 4.89 | 4.39 | 1.25 | 1.11 |
| **1** | 2.32 | 3.36 | 2.41 | 0.64 | 0.91 | 3.10 | 4.35 | 4.00 | 1.45 | 1.25 |
| **2** | 2.50 | 3.33 | 2.67 | 1.08 | 1.25 | 1.70 | 3.05 | 2.75 | 1.05 | 0.55 |
| **3** | 2.29 | 2.21 | 2.25 | 0.67 | 0.50 | 2.15 | 2.50 | 1.55 | 1.35 | 2.35 |
| **4** | 2.75 | 3.50 | 2.42 | 1.17 | 0.58 | - | - | - | - | - |

| | | | | | | | | | | |
|---|---|---|---|---|---|---|---|---|---|---|
| ¹Example 14 fragranced with 85% (w/w) of Floral-1 (Samples 1-3) and Fresh-1 (Samples 4-6) fragrances. ²EVA Floral-1 product (Samples A&B) and EVA Fresh-1 (Samples C&D) product. | | | | | | | | | | |

**Table 5**

| **Intensity Profile of Example 14 vs. Scented Polymer** | | | | | | | | | | |
|---|---|---|---|---|---|---|---|---|---|---|
| **Week** | **Trial 1** | | | | | **Trial 2** | | | | |
| | **Fragranced Samples¹** | | | **Scented Polymer²** | | **Fragranced Samples¹** | | | **Scented Polymer²** | |
| | **1** | **2** | **3** | **A** | **B** | **4** | **5** | **6** | **C** | **D** |
| **0** | 4.67 | 4.50 | 4.22 | 2.83 | 2.50 | 4.58 | 4.83 | 4.50 | 2.00 | 2.13 |
| **1** | 2.22 | 3.06 | 2.89 | 1.83 | 2.28 | 1.94 | 3.69 | 3.50 | 1.43 | 1.56 |
| **2** | 2.75 | 2.56 | 1.63 | 0.63 | 0.81 | 3.21 | 2.57 | 2.57 | 1.43 | 1.50 |
| **3** | 1.94 | 2.33 | 1.67 | 0.78 | 0.78 | 1.19 | 2.31 | 1.81 | 0.63 | 0.50 |
| **4** | 2.44 | 1.94 | 1.28 | 0.44 | 1.00 | 2.35 | 2.05 | 1.25 | 0.80 | 0.70 |

| | | | | | | | | | | |
|---|---|---|---|---|---|---|---|---|---|---|
| ¹Example 14 fragranced with 85% (w/w) of Citrus-1 (Samples 1-3) and Fresh-1 (Samples 4-6) fragrances. ²Scented polymer product with Citrus-1 (Samples A&B) and Fresh-1 (Sample C&D) fragrance. | | | | | | | | | | |

**Table 6**

| **Intensity Profile of Example 14 vs. Scented Paper** | | | | | | | | | | |
|---|---|---|---|---|---|---|---|---|---|---|
| **Week** | **Trial 1** | | | | | **Trial 2** | | | | |
| | **Fragranced Samples¹** | | | **Scented Paper²** | | **Fragranced Samples¹** | | | **Scented Paper²** | |
| | **1** | **2** | **3** | **A** | **B** | **4** | **5** | **6** | **C** | **D** |
| **0** | 3.26 | 3.83 | 2.90 | 4.17 | 4.31 | 4.44 | 4.69 | 4.56 | 4.69 | 4.50 |
| **1** | 3.44 | 3.31 | 1.88 | 2.13 | 2.38 | 3.35 | 3.50 | 2.70 | 1.85 | 2.20 |
| **2** | 3.06 | 2.33 | 2.33 | 0.50 | 0.83 | 2.55 | 2.70 | 2.50 | 2.30 | 1.70 |
| **3** | 1.29 | 1.96 | 1.33 | 0.88 | 0.79 | 2.80 | 2.70 | 1.65 | 0.95 | 1.20 |
| **4** | 2.00 | 1.95 | 1.95 | 1.00 | 0.86 | - | - | - | - | - |

| | | | | | | | | | | |
|---|---|---|---|---|---|---|---|---|---|---|
| ¹Example 14 fragranced with 85% (w/w) of Floral-1 (Samples 1-3) and Fresh-1 (Samples 4-6) fragrances. ²Scented paper product with Floral-1 (Samples A&B) and Fresh-1 (Samples C&D) fragrance. | | | | | | | | | | |

**Table 7**

| **Intensity Profile of Example 14 vs. Scented Paper** | | | | | | | | | | |
|---|---|---|---|---|---|---|---|---|---|---|
| **Week** | **Trial 3** | | | | | **Trial 4** | | | | |
| | **Fragranced Samples** | | | **Scented Paper** | | **Fragranced Samples** | | | **Scented Paper** | |
| | **7** | **8** | **9** | **E** | **F** | **10** | **11** | **12** | **G** | **H** |
| **0** | 3.89 | 3.61 | 2.78 | 4.44 | 4.17 | 4.38 | 3.94 | 3.31 | 4.56 | 4.50 |
| **2** | 1.21 | 1.29 | 1.29 | 2.00 | 2.36 | 2.85 | 3.88 | 3.42 | 1.69 | 2.35 |
| **3** | 1.13 | 2.00 | 1.87 | 0.90 | 0.87 | 1.47 | 1.94 | 2.44 | 1.41 | 1.13 |
| **4** | 1.32 | 1.39 | 1.34 | 0.45 | 0.55 | 0.93 | 1.30 | 1.78 | 2.00 | 1.45 |

| | | | | | | | | | | |
|---|---|---|---|---|---|---|---|---|---|---|
| ¹Example 14 fragranced with 85% (w/w) of Floral-1 (Samples 7-9) and Fresh-1 (Samples 10-12) fragrances. ²Scented paper product with Floral-1 (Samples E&F) and Fresh-1 (Samples G&H) fragrance. | | | | | | | | | | |

**Table 8**

| **Intensity Profile of Example 14 vs. Auto Vent Product** | | | | | | | | | | | | |
|---|---|---|---|---|---|---|---|---|---|---|---|---|
| **Week** | **Trial 1** | | | | | | **Trial 2** | | | | | |
| | **Fragranced Samples¹** | | | **Auto Vent Product²** | | | **Fragranced Samples¹** | | | **Auto Vent Product²** | | |
| | **1** | **2** | **3** | **A** | **B** | **C** | **4** | **5** | **6** | **D** | **E** | **F** |
| **0** | 5.13 | 5.34 | 5.18 | 5.21 | 5.18 | 4.92 | 5.63 | 5.79 | 5.55 | 5.29 | 5.71 | 5.21 |
| **1** | 4.75 | 4.5 | 4.91 | 5.16 | 5.28 | 4.41 | 5.25 | 5.36 | 5.86 | 5.39 | 5.43 | 5.93 |
| **2** | - | - | - | - | - | - | 4.56 | 4.75 | 5.41 | 5.38 | 5.31 | 5.75 |
| **3** | 4.89 | 4.89 | 4.92 | 4.86 | 5.19 | 3.75 | 5.50 | 4.33 | 5.92 | 5.83 | 6.17 | 6.14 |
| **4** | 4.42 | 4.46 | 4.96 | 4.46 | 5.21 | 3.33 | 4.68 | 4.75 | 5.46 | 5.57 | 5.57 | 5.89 |

| | | | | | | | | | | | | |
|---|---|---|---|---|---|---|---|---|---|---|---|---|
| ¹Example 14 fragranced with 85% (w/w) of Floral-1 (Samples 1 & 4), Woody-1 (Samples 2 & 5), & Fresh-2 (Samples 3 & 6) fragrances. ²Auto vent product with Floral-2 (Sample A & D), Woody-2 (Samples B & E) & Fresh-3 (Samples C & F) fragrances, used as a comparison. | | | | | | | | | | | | |

In view of the above, it will be seen that the several objects of the disclosure are achieved, and other advantageous results attained.

As is evident from the foregoing description, certain aspects of the present invention is not limited by the particular details of the examples illustrated herein, and it is therefore contemplated that other modifications, applications, variations, or equivalents thereof, will occur to those skilled in the art. Many such changes, modifications, variations and other uses and applications of the present constructions will, however, become apparent to those skilled in the art after considering the specification and the accompanying drawings. In addition, unless mention was made above to the contrary, it should be noted that all of the accompanying drawings are not to scale. All such changes, modifications, variations and other uses in applications which do not depart from the spirit and scope of the present inventions are deemed to be covered by the inventions which are limited only by the claims which follow.

## Claims

1. A fragrance composition comprising
(1) a polymer substrate comprising the reaction product of an acrylate monomer and an oligomer crosslinker having at least two arms; and
(2) a fragrance.

2. The fragrance composition of claim 1 wherein the acrylate monomer further comprises an alkyl group.

3. The fragrance composition of claim 2 wherein the alkyl group comprises at least one carbon and at most about ten carbons.

4. The fragrance composition of any one of claims 1 to 3 wherein the acrylate monomer is provided as methyl acrylate.

5. The fragrance composition of any one of claims 1 to 4 wherein the oligomer crosslinker comprises an acrylate crosslinker.

6. The fragrance composition of any one of claims 1 to 5 wherein the oligomer crosslinker comprises three arms.

7. The fragrance composition of any one of claims 1 to 4 wherein the oligomer crosslinker comprises TMPTMA.

8. The fragrance composition of any one of claims 1 to 7 wherein the mass ratio of the oligomer crosslinker to the acylate monomer used to form the polymer substrate is from about 1:2 to about 1:50.

9. The fragrance composition of claim 7 wherein the mass ratio of the oligomer crosslinker to the acylate monomer used to form the polymer substrate is from about 1:2 to about 1:10.

10. The fragrance composition of any one of claims 1 to 9 wherein the mole ratio of the oligomer crosslinker and the monomer used to form the polymer substrate is from about 1:7 to about 1:10.

11. The fragrance composition of any one of claims 1 to 10 wherein the mole ratio of the oligomer crosslinker and the monomer used to form the polymer substrate is no more than 1:8.

12. The fragrance composition of any one of claims 1 to 11 comprising the fragrance in a concentration of at least about 45% by weight, relative to the concentration of the fragrance composition as a whole.

13. The fragrance composition of any one of claims 1 to 12 comprising the polymer substrate in a concentration of at most about 60% by weight, relative to the concentration of the fragrance composition as a whole.

14. The fragrance composition of any one of claims 1 to 13 comprising the fragrance in a concentration of about 40% by weight to about 75 % by weight, relative to the composition as a whole.

15. A method of preparing a fragrance composition, the method comprising:
(1) a dissolution step, wherein the monomer and the oligomer crosslinker are dissolved in an organic solvent, thereby forming a monomer-crosslinker solution;
(2) a polymerization step, wherein the monomer is contacted with the oligomer crosslinker in the presence of an initiator to form a polymer substrate; and
(3) a fragrance encapsulation step, wherein a fragrance is introduced to and encapsulated in the polymer substrate.

16. The method of claim 15 further comprising (4) an extraction step, wherein the polymer substrate is extracted from the reaction mixture and (5) a washing step wherein the polymer substrate is purified.

17. The method as in any of claims 15 or 16 wherein the organic solvent comprises a polar organic solvent.

18. The method as in any one of claims 15 to 17 wherein the initiator comprises an azo-type initiator.

19. The method as in any one of claims 15 to 18 wherein the polymerization step is carried out at a temperature of about 60 □ to about 80 □.

20. The method as in any one of claims 15 to 19 wherein the polymer substrate has a residual percentage of no more than about 5% by weight, relative to the composition as a whole.
